# EUROPÄISCHE PATENTSCHRIFT

(11) **EP 1 292 354 B1**
(45) Veröffentlichungstag und Bekanntmachung des Hinweises auf die Patenterteilung: **16.06.2010**
(21) Anmeldenummer: 01980026.7
(22) Anmeldetag: 22.05.2001
(51) Int. Cl.: A61M 25/00

(54) **EIN MIT WEICHER SPITZE AUSGEFÜHRTER KATHETER ZUR STENTIMPLANTATION**
SOFT TIP CATHETER SYSTEM FOR USE IN STENT IMPLANTATION
ENSEMBLE CATHETER À EMBOUT SOUPLE POUR IMPLANTATION D'UN STENT

(30) Priorität: 22.05.2000 DE 20009204 U
(43) Veröffentlichungstag der Anmeldung: 19.03.2003
(73) Patentinhaber: Abbott Laboratories Vascular Enterprises Limited, Dublin 2 (IE)
(72) Erfinder: JÖRGENSEN, Ib, 72401 Haigerloch (DE); VON OEPEN, Randolf, 72074 Tübingen (DE)
(74) Vertreter: Schmitz, Hans-Werner
(86) Internationale Anmeldenummer: PCT/EP2001/005893
(87) Internationale Veröffentlichungsnummer: WO 2001/089620

(56) Entgegenhaltungen:
- EP-A- 0 277 368
- WO-A-99/44666
- US-A- 5 209 729
- US-A- 5 334 148
- US-A- 5 425 712
- US-A- 5 549 552
- US-A- 5 728 063

## Beschreibung

Die vorliegende Erfindung betrifft eine Katheteranordnung nach dem Oberbegriff des Anspruchs 1. Eine derartige Katheteramordung ist aus der WO 99 44666 A bekannt.

An Verengungsstellen in Körpergefäßen oder Körperhöhlungen werden heutzutage zur Aufweitung der Verengung Katheteranordnungen verwendet, welche an ihrem distalen Ende einen Ballon aufweisen, welcher expandierbar ist. Auf diesen expandierbaren Ballon kann zusätzlich ein Stent aufgecrimpt sein, der zur Stabilisierung der Gefäßwand in der Verengung platziert werden kann. Die Katheteranordnung wird dann mit Hilfe eines Führungsdrahtes an die verengte Stelle im Körper des Patienten geführt und die Verengung durch Expansion des Ballons aufgeweitet beziehungsweise der aufgecrimpte Stent in der Verengung platziert.

Es ist daher Aufgabe der vorliegenden Erfindung eine Katheteranordnung der im Oberbegriff des Anspruchs 1 angegebenen Art zu schaffen, welche eine besonders hohe Flexibilität am distalen Endbereich aufweist.

Die Lösung dieser Aufgabe erfolgt durch die Merkmale des Anspruchs 1. Eine vorteilhafte Weiterbildung ist Gegenstand des Unteranspruches.

Die Qualität eines Ballonkatheters zeichnet sich dadurch aus, dass der Katheter möglichst einfach gewundenen Gefäßabschnitten folgen kann. Wird daher bevorzugend die distale Spitze - im Allgemeinen "Tip" genannt - aus einem hierfür besonders geeigneten biegeweicheren Material gestaltet, so folgt das gesamte System bevorzugt den Windungen des Gefäßes. Gleichzeitig muss jedoch gewährleistet sein, dass der Katheter in die verengten Bereiche des Gefäßes sicher vorgeschoben werden kann. Diese Eigenschaft wird durch den Fachbegriff "pushability" beschrieben. Sofern jedoch das gesamte Kathetersystem aus weichen Materialien gefertigt wird, wirkt dies kontraindikativ.

Erfindungsgemäss ist der Führungsdrahttubus aus zwei zusammengesetzten Teilen hergestellt, wobei die beiden Teile aus unterschiedlichen Materialien bestehen. Dabei ist der Teil des Führungsdrahttubus, welcher das distale Ende bildet, aus einem Material mit einer größeren Elastizität bzw. Flexibilität als der andere Teil hergestellt. Dadurch kann eine besonders flexible Ausgestaltung des distalen Endes des Führungsdrahttubus bzw. der Katheteranordnung erreicht werden. Die Katheteranordnung erhält dadurch eine größere Biegefähigkeit unter gleichzeitiger Erhaltung der "pushability".

Zur Verbindung der beiden Teile des Führungsdrahttubus sind u.a. Methoden wie Kleben und Schweißen (Laser-, Kontakt- und Heißluft-Schweißen sowie induktives Schweißen) denkbar.

Gemäß der vorliegenden Erfindung ist ein Übergangsbereich des Führungsdrahttubus von dem aus einem ersten Material hergestellten Teil zu dem aus einem zweiten Material hergestellten Teil auf der distalen Seite des Ballons angeordnet. Dadurch ist die distale Befestigungsposition des Ballons vor dem Übergangsbereich des Führungsdrahttubus angeordnet.

Weitere Einzelheiten, Merkmale und Vorteile der Erfindung ergeben sich aus der nachfolgenden Beschreibung von Ausführungsbeispielen anhand der Zeichnung.

Es zeigt:
- Fig. 1: eine schematisch stark vereinfachte Schnittdarstellung des prinzipiellen Aufbaues einer erfindungsgemäßen Katheteranordnung ; und
- Fig. 2: eine schematisch stark vereinfachte Schnittdarstellung einer erfindungsgemäßen Katheteranordnung gemäß der vorliegenden Erfindung.

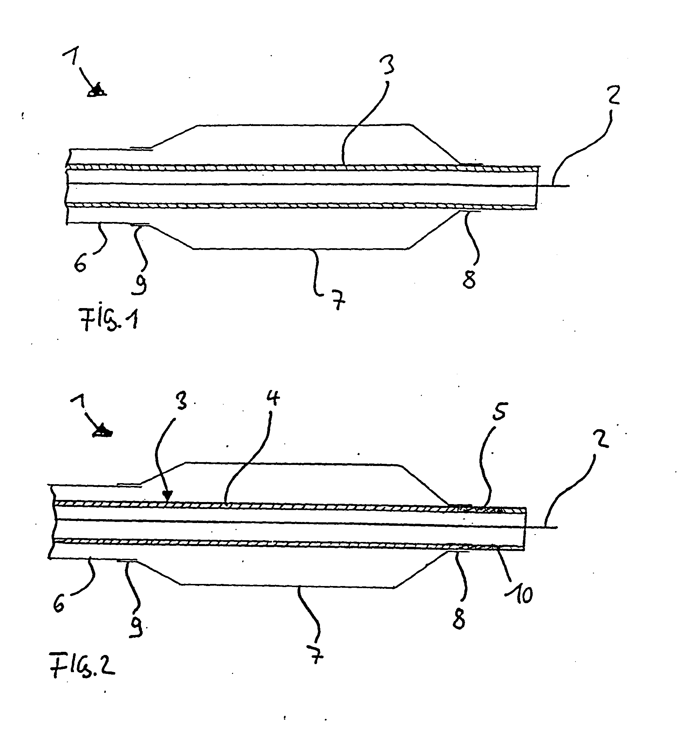

In Fig. 1 ist eine Katheteranordnung 1 zur Implantation eines Stents dargestellt. Die Katheteranordnung 1 weist eine Führungsdrahttubus 3 auf, in dem ein Führungsdraht 2 angeordnet ist. Der Führungsdraht 2 und der Führungsdrahttubus 3 bilden gemeinsam eine Führungseinrichtung.

Wie in Fig. 1 gezeigt, umfasst die Katheteranordnung 1 weiter einen Ballon 7 und einen Tubus 6. Der Ballon 7 ist mit seinem distalen Ende über einen distalen Befestigungsbereich 8 am Führungsdrahttubus 3 befestigt und mit seinem proximalen Ende über einen proximalen Befestigungsbereich 9 am Tubus 6 befestigt (vgl. Fig. 1). Hierbei wird unter "distalem Ende" "zum Herzen hinführend" und unter "proximalem Ende" "vom Herzen wegführend" verstanden. Der mit dem Ballon 7 verbundene Tubus 6 dient dazu, um ein geeignetes Medium wie z.B. eine Kochsalzlösung in den hohlen Innenraum des Ballons 7 zuzuführen und diesen an der verengten Stelle zu expandieren. Dadurch kann ein um den Ballon 7 angebrachter Stent (nicht dargestellt) expandiert werden und seinerseits die Aufweitung der verengten Stelle im Körperhohlgefäß als Gefäßstütze stabilisieren.

Wie aus Fig. 1 ersichtlich ist, ist der distale Befestigungsbereich 8 des Ballons 7 am Führungsdrahttubus 3 etwas in proximaler Richtung vom distalen Ende des Führungsdrahttubus versetzt angeordnet. Dadurch ist die Verbindungsstelle zwischen Ballon 7 und Führungsdrahttubus 3 versetzt vom Ende des Führungsdrahttubus 3 angeordnet.

Die erfindungsgemäße Katheteranordnung weist im Gegensatz zu bekannten Katheteranordnungen einen weichen Spitzenbereich auf, dessen Flexibilität beziehungsweise Elastizität größer ist als der verbleibende Teil des Führungsdrahttubus. Dadurch können Verletzungen des Patienten beim Einführen der Katheteranordnung verhindert-werden.

In Fig. 2 ist ein Ausführungsbeispiel der Katheteranordnung gemäß der vorliegenden Erfindung dargestellt. Gleiche Teile sind mit gleichen Bezugszeichen wie im ersten Ausführungsbeispiel bezeichnet.

Die Katheteranordnung gemäss Fig. 2 weist einen Führungsdrahttubus 3 auf, welcher aus zwei Teilen 4, 5 besteht. Hierbei sind die beiden Teile 4, 5 aus unterschiedlichen Materialien hergestellt. Das Teil 5 des Führungsdrahttubus 3, welches das distale Ende des Führungsdrahttubus 3 bildet, ist dabei aus einem elastischeren Material hergestellt als das Teil 4 des Führungsdrahttubus 3. Somit weist das distale Ende des Führungsdrahttubus 3 eine hohe Flexibilität beziehungsweise Elastizität auf. Dies wird noch dadurch verstärkt, dass der distale Befestigungsbereich 8, an welchem der Ballon 7 mit dem Führungsdrahttubus 3 verbunden ist, vom Ende des Führungsdrahttubus 3 versetzt angeordnet ist.

Wie in Fig. 2 gezeigt, ist hierbei ein Übergangsbereich 10 zwischen den beiden Teilen 4 und 5 des Führungsdrahttubus auf der distalen Seite des Befestigungsbereiches 8 angeordnet.

## Patentansprüche

1. Katheteranordnung (1)
- mit einem Führungsdrahttubus (3) zur Aufnahme eines Führungsdrahtes (2),
- mit einem expandierbaren Ballon (7), der mit seinem distalen Ende über einen distalen Befestigungsbereich (8) am Führungsdrahttubus (3) und mit seinem proximalen Ende über einen proximalen Befestigungsbereich (9) an einem Tubus (6) zur Zuführung von Expansionsmedium zum Inneren des Ballons (7) befestigt ist, wobei das distale Ende des Führungsdrahttubus (3) über das distale Ende des Ballons (7) hinaussteht und als weiche Spitze ausgebildet ist, und wobei ein erster Teil (4) des Führungsdrahttubus (3) aus einem ersten Material und ein zweiter Teil (5) des Führungsdrahttubus (3) aus einem zweiten Material größerer Flexibilität besteht und ein Übergangsbereich (10) vom ersten Teil (4) zum zweiten Teil (5) vorgesehen ist, und
- mit einen Stent, der um den Ballon (7) herum angebracht ist,
**dadurch gekennzeichnet,**
- **dass** der Übergangsbereich (10) des Führungsdrahttubus (3) auf der distalen Seite des Befestigungsbereichs (8) des Ballons (7) angeordnet ist.

2. Katheteranordnung nach Anspruch 1, **dadurch gekennzeichnet, dass** die Länge des distalen Endes des Führungsdrahttubus (3), welche aus dem Ballon (7) in Richtung des Führungsdrahts (2) hervorsteht, größer ist als eine Länge des distalen Befestigungsbereichs (8) zwischen dem Ballon (7) und dem Führungsdrahttubus (3).

## Claims

1. A catheter arrangement (1), comprising
- a guide wire tube (3) for receiving a guide wire (2),
- an expandable balloon (7), the distal end whereof is fastened to said guide wire tube (3) through a distal fastening portion (8) and the proximal end whereof is fastened to a tube (6) through a proximal fastening portion (9) for supplying an expansion medium to the interior of the balloon (7), the distal end of the guide wire tube (3) projecting beyond the distal end of the balloon (7) and being formed as a soft tip, and a first part (4) of the guide wire tube (3) being made of a first material and a second part (5) of the guide wire tube (3) being made of a second material having a higher flexibility, and a transition portion (10) being provided from the first part (4) to the second part (5), and
- a stent arranged around the balloon (7),
**characterized in that**
- the transition portion (10) of the guide wire tube (3) is arranged on the distal side of the fastening portion (8) of the balloon (7).

2. The catheter arrangement of claim 1, **characterized in that** the length of the distal end of the guide wire tube (3) projecting from the balloon (7) toward the guide wire (2) is larger than a length of the distal fastening portion (8) between the balloon (7) and the guide wire tube (3).

## Revendications

1. Ensemble de cathéter (1) comportant
- un tube de fil-guide (3) pour recevoir un fil-guide (2),
- un ballonnet expansible (7), qui est fixé avec son extrémité distale, par le biais d'une zone de fixation distale (8) au tube de fil-guide (3) et avec son extrémité proximale, par le biais d'une zone de fixation proximale (9) à un tube (6) pour le guidage d'un milieu d'expansion à l'intérieur du ballonnet (7), l'extrémité distale du tube de fil-guide (3) dépassant sur l'extrémité distale du ballonnet (7) et étant configurée sous la forme d'une pointe souple, une première partie (4) du tube de fil-guide (3) étant constituée d'un premier matériau et une deuxième partie (5) du tube de fil-guide (3) étant constituée d'un deuxième matériau présentant une flexibilité supérieure et une zone de transition (10) de la première partie (4) à la deuxième partie (5) étant prévue, et
- un stent qui est placé autour du ballonnet (7),
**caractérisé en ce que**
- la zone de transition (10) du tube de fil-guide (3) est disposée sur le côté distal de la zone de fixation (8) du ballonnet (7).

2. Ensemble de cathéter selon la revendication 1, **caractérisé en ce que** la longueur de l'extrémité distale du tube de fil-guide (3) qui dépasse du ballonnet (7) dans le sens du fil-guide (2), est supérieure à une longueur de la zone de fixation distale (8) entre le ballonnet (7) et le tube de fil-guide (3).
